# EUROPÄISCHE PATENTSCHRIFT

(11) **EP 4 277 596 B1**
(45) Veröffentlichungstag und Bekanntmachung des Hinweises auf die Patenterteilung: **12.03.2025**
(21) Anmeldenummer: 22700503.0
(22) Anmeldetag: 14.01.2022
(51) Int. Cl.: A61K 9/00

(54) **MEHRSCHICHTIGER ORALER DÜNNFILM**
MULTI-LAYER ORAL THIN FILM
FILM MINCE ORAL MULTICOUCHE

(30) Priorität: 15.01.2021 DE 102021100783
(43) Veröffentlichungstag der Anmeldung: 22.11.2023
(73) Patentinhaber: LTS Lohmann Therapie-Systeme AG, 56626 Andernach (DE)
(72) Erfinder: LINN, Michael, 55596 Waldböckelheim (DE); SCHMITZ, Christoph, 56598 Rheinbrohl (DE); NORELLI, Claudia, 56204 Hillscheid (DE); FICKER, Mario, 53179 Bonn (DE)
(74) Vertreter: Held, Stephan
(86) Internationale Anmeldenummer: PCT/EP2022/050800
(87) Internationale Veröffentlichungsnummer: WO 2022/152883

(56) Entgegenhaltungen:
- WO-A1-2009/124096
- MASEK JOSEF ET AL: "Multi-layered nanofibrous mucoadhesive films for buccal and sublingual administration of drug-delivery and vaccination nanoparticles - important step towards effective mucosal vaccines", JOURNAL OF CONTROLLED RELEASE, ELSEVIER, AMSTERDAM, NL, vol. 249, 25 July 2016 (2016-07-25), pages 183 - 195, XP029927819, ISSN: 0168-3659, DOI: 10.1016/J.JCONREL.2016.07.036
- YOGYATA S PATHARE ET AL: "Polymers used for Fast Disintegrating Oral Films: A Review", INTERNATIONAL JOURNAL OF PHARMACEUTICAL SCIENCES REVIEW AND RESEARCH, 1 July 2013 (2013-07-01), pages 169 - 178, XP055566414, Retrieved from the Internet <URL:http://globalresearchonline.net/journalcontents/v21-1/29.pdf> [retrieved on 20190308]

## Beschreibung

Die vorliegende Erfindung betrifft einen mehrschichtigen oralen Dünnfilm, ein Verfahren zu dessen Herstellung, sowie den mehrschichtigen oralen Dünnfilm zur Verwendung als Arzneimittel.

Orale Dünnfilme sind dünne, mindestens einen pharmazeutisch aktiven Wirkstoff enthaltende Filme, die direkt in den Mundraum gelegt oder an die Mundschleimhaut angelegt werden und sich dort auflösen bzw. aufquellen und dabei den Wirkstoff abgeben. Dabei handelt es sich insbesondere um dünne, ein- oder mehrschichtige, wirkstoffhaltige Filme auf Polymerbasis, die, wenn sie auf die Schleimhaut, insbesondere die Mundschleimhaut, aufgebracht werden, den Wirkstoff direkt in diese abgeben können. Die sehr gute Durchblutung der Mundschleimhaut sorgt für einen schnellen Übergang des Wirkstoffs in den Blutkreislauf. Dieses Darreichungssystem hat den Vorteil, dass der Wirkstoff größtenteils durch die Schleimhaut resorbiert wird und damit der "First-Pass Metabolismus", der bei der konventionellen Darreichungsform eines Wirkstoffs in Tablettenform auftritt, vermieden wird. Der Wirkstoff kann in dem Film gelöst, emulgiert oder dispergiert werden.

Mehrschichtige orale Dünnfilme können gegenüber "normalen" einschichtigen oralen Dünnfilmen diverse Vorteile bieten. Z.B. kann durch die Kombination mehrerer Schichten ein hohes Flächengewicht erreicht werden und es können verschiedene Schichten kombiniert werden, wie z.B. eine Wirkstoffschicht mit einer pH regulierenden Schicht, oder eine Wirkstoffschicht mit einer schützenden Rückschicht.

Mašek Josef, et al. Multi-layered nanofibrous mucoadhesive films for buccal and sublingual administration of drug-delivery and vaccination nanoparticles - important step towards effective mucosal vaccines. J Control Release. 10. März 2017; Seiten 183-195, beschreibt Nanofaser-basierte mukoadhäsive Filme wurden für die oromukosale Verabreichung von Nanocarriern zur Verabreichung von Medikamenten und Impfstoffen entwickelt.

Yogyata S Pathare et al. "Polymers used for Fast Disintegrating Oral Films: A Review", International J. of Pharmaceutical Sciences Review and Research, 1. Juli 2013, Seiten 169-178, beschreibt die aktuellen Entwicklungen im Bereich der oralen Dünnschicht als neues Verabreichungssystem für die orale Verabreichung von Medikamenten.

WO 2009/124096 A1 offenbart ein Pflaster, ein System und ein Verfahren zur Verabreichung einer permeanten Zusammensetzung in ein Subjekt über mindestens einen gebildeten Weg durch eine biologische Membran des Subjekts.

Mehrschichtige orale Dünnfilms können prinzipiell auf verschiedenen Wegen hergestellt werden, wie z.B. durch Mehrfachbeschichtung oder durch das Zusammenkleben bzw. Zusammenlaminieren von vorgefertigten Schichten.

Die Mehrfachbeschichtung weist hier diverse Nachteile auf. So wird beispielsweise der Wirkstoff durch mehrfache Beschichtungsgänge stärker thermisch belastet. Ferner ist ein solcher Verbund zwischen den Schichten häufig nicht stark und kann wieder delaminieren. Außerdem pflanzen sich Fehler in der Beschichtung (Flächengewicht) in die folgenden Schichten fort.

Auch das Zusammenfügen mehrerer Schichten mittels Zusammenkleben weist Nachteile auf. Bekannte Klebeschichten weisen oft eine hohe Dichte an funktionalen Gruppen, wie -OH-Gruppen, auf, welche die Migration von Wirkstoff bzw. von Hilfsstoffen wie Puffersalzen durch die Klebeschicht begünstigen. Zudem sind bekannte Klebeschichten häufig schlecht wasserlöslich und zeigen damit ein schlechtes Auflöseverhalten im Mund des Patienten (schlechtes Mundgefühl). Außerdem ist in den bekannten Klebeschichten häufig der Einsatz von großen Mengen Weichmachern (z.B. 20% Glycerin) nötig, welche in die anderen Schichten des oralen Dünnfilms migrieren können und dort die physikalischen Eigenschaften verändern (z.B. Absenkung der Glasübergangstemperatur).

Die Aufgabe der vorliegenden Erfindung war es die aus dem Stand der Technik bekannten Nachteile zu beheben. Insbesondere soll ein mehrschichtiger oraler Dünnfilm bereitgestellt werden, bei dem die einzelnen Schichten fest zusammengefügt werden, wobei auf die bekannten Klebeschichten und/oder auf das Aufeinanderbeschichten verzichtet werden kann. Ferner soll eine Migration von Stoffen zwischen den einzelnen Schichten weitgehend verhindert werden. Außerdem soll der mehrschichtige orale Dünnfilm möglichst leicht und kostengünstig hergestellt werden können.

Diese Aufgaben wurden nun durch einen oralen Dünnfilm gemäß Anspruch 1 gelöst, d.h. durch einen mehrschichtigen oraler Dünnfilm, umfassend eine erste und eine zweite Matrixschicht, die jeweils mindestens ein Polymer enthalten, und eine zwischen der ersten und der zweiten Matrixschicht befindliche Trennschicht, wobei die Trennschicht mindestens ein Polyethylenglycol mit einem Molekulargewicht von 8000 g/mol bis 7000000 g/mol in einer Menge von 60 bis 100 Gew.-% umfasst.

Ein solcher mehrschichtiger oraler Dünnfilm hat u.a. den Vorteil, dass sich Polyethylenglycol-Filme aufgrund ihrer glatten Oberfläche, ihrer niedrigen Schmelz- bzw. Glasübergangstemperatur, ihrer unbedenklicher Toxizität und ihrer Wasserlöslichkeit gut als Trennschichten/Verbindungsschichten/Klebeschichten in mehrschichtigen oralen Dünnfilmen eignen. Durch Erwärmen und/oder hohen Druck eignen sich Polyethylenglycol-Klebeschichten insbesondere dazu zwei weitere Schichten miteinander zu verbinden. Dabei können beispielsweise eine wirkstoffhaltige Schicht und eine pH-regulierende Puffer-Schicht miteinander verbunden werden.

Prinzipiell können mehrere Laminate, auch mit verschiedenen Wirkstoffen/Hilfsstoffen, zu einem "Sandwich-OTF" zusammengesetzt werden.

Des Weiteren eignet sich die Polyethylenglycol-Schicht als Sperrschicht, welche die Migration von Wirkstoffen oder Hilfsstoffen (z.B. Puffersalzen) zwischen den einzelnen Schichten verhindert bzw. minimiert.

Die Polyethylenglycol-Klebeschicht benötigt zudem keine oder nur geringe Mengen Hilfsstoffe wie Weichmacher (z.B. 2-3% Glycerin) und reduziert somit das Risiko einer Migration der Klebeschichtbestandteile in die anderen Schichten des oralen Dünnfilms.

In der vorliegenden Schrift kann "umfassend" auch "bestehend aus" bedeuten.

Im Folgenden sind die Begriffe "Trennschicht", "Verbindungsschicht" und "Klebeschicht" gleichwertig zu verstehen.

Polyethylenglycole (PEG) sind Verbindungen der allgemeinen Formel:

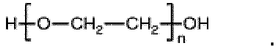

Höhermolekulare feste Polyethylenglycole (Schmelztemperatur etwa 65 °C) werden häufig auch als Polyethylenoxide oder auch Polyoxyethylene (Kurzzeichen PEO oder seltener PEOX) oder Polywachse bezeichnet. In der vorliegenden Schrift werde die Begriffe "Polyethylenglycol", "Polyethylenoxid" und "PolyOx"gleichwertig verwendet.

Die folgenden Definitionen bezüglich der ersten und der zweiten Matrixschicht gelten immer analog für beide, die erste und die zweite Matrixschicht.

Prinzipiell können die erste und die zweite Matrixschicht eine gleiche Zusammensetzung oder eine unterschiedliche Zusammensetzung aufweisen.

Der erfindungsgemäße mehrschichtige orale Dünnfilm ist vorzugsweise dadurch gekennzeichnet, dass die erste und/oder die zweite Matrixschicht mindestens ein wasserlösliches Polymer umfasst.

Wasserlösliche Polymere umfassen chemisch sehr unterschiedliche, natürliche oder synthetische Polymere, deren gemeinsames Merkmal ihre Löslichkeit in Wasser oder wässrigen Medien ist. Voraussetzung ist, dass diese Polymere eine für die Wasserlöslichkeit ausreichende Anzahl an hydrophilen Gruppen besitzen und nicht vernetzt sind. Die hydrophilen Gruppen können nicht-ionisch, anionisch, kationisch und/oder zwitterionisch sein.

Unter wasserlöslich wird vorzugsweise eine Löslichkeit von größer als 100 g/L in Wasser bei 25°C verstanden.

Das mindestens eine wasserlösliche Polymer ist vorzugsweise ausgewählt aus der Gruppe bestehend aus Stärke und Stärkederivaten, Dextranen, Cellulosederivaten, wie Carboxymethylcellulose, Hydroxypropylcellulose, Hydroxyethylcellulose, Hydroxypropylmethylcellulose, Hydroxypropylethylcellulose, Natriumcarboxymethylcellulose, Ethyl- oder Propylcellulose, Polyacrylsäuren, Polyacrylaten, Polyvinylpyrrolidonen, Vinvlpyrrolidon/Vinylacetat-Copolymeren, Polyvinylalkoholen, Polyethylenoxidpolymeren, Polyacrylamiden, Polyethylenglycolen, Gelatine, Collagen, Alginaten, Pektin, Pullulan, Traganth, Chitosan, Alginsäure, Arabinogalaktan, Galaktomannan, Agar, Agarose, Carrageen und natürlichen Gummen, wobei Polyvinylalkohole besonders bevorzugt sind.

Der erfindungsgemäße mehrschichtige orale Dünnfilm ist vorzugsweise dadurch gekennzeichnet, dass das mindestens eine Polymer, vorzugsweise das wasserlösliche Polymer, in einer Menge von 10 bis 90 Gew.-%, vorzugsweise von 20 bis 60 Gew.-%, besonders bevorzugt von 30 bis 50 Gew.-%, bezogen auf das Gesamtgewicht der ersten und/oder der zweiten Matrixschicht, in der jeweiligen Matrixschicht vorhanden ist.

Die erste und/oder die zweite Matrixschicht enthält vorzugsweise mindestens einen pharmazeutisch aktiven Wirkstoff.

Die erste und zweite Matrixschicht können einen gleichen oder einen unterschiedlichen pharmazeutisch aktiven Wirkstoff enthalten.

Der mindestens eine pharmazeutisch aktive Wirkstoff unterliegt prinzipiell keiner Beschränkung, ist aber vorzugsweise ausgewählt aus allen pharmazeutisch aktiven Wirkstoffen, die zur oralen und/oder transmucosalen Applikation geeignet sind.

Gemäß der vorliegenden Erfindung werden unter dem pharmazeutisch aktiven Wirkstoff auch alle pharmazeutisch akzeptablen Salze und Solvate des jeweiligen pharmazeutisch aktiven Wirkstoffs subsumiert.

Bevorzugt sind Wirkstoffe ausgewählt aus der Gruppe, umfassend die Wirkstoffklassen der Analgetika, Hormone, Hypnotika, Sedativa, Antiepileptika, Weckamine, Psychoneurotropika, Neuro-Muskelblocker, Antispasmodika, Antihistaminika, Antiallergika, Cardiotonika, Antiarrhythmika, Diuretika, Hypotensiva, Vasopressoren, Antidepressiva, Antitussiva, Expectorantia, Thyroidhormone, Sexualhormone, Antidiabetika, Antitumor-Wirkstoffe, Antibiotika, Chemotherapeutika und Narcotika, wobei diese Gruppe nicht abschließend ist.

Besonders bevorzugt handelt es sich bei dem mindestens einen pharmazeutisch aktiven Wirkstoff um Ketamin und/oder ein pharmazeutisch akzeptables Salz oder Solvat davon, bevorzugt Ketamin·HCl.

Unter Ketamin wird (S)-(±)-2-(2-Chlorphenyl)-2-(methylamino)cyclohexan-1-on, (R)-(±)-2-(2-Chlorphenyl)-2-(methylamino)cyclohexan-1-on, sowie das Racemat (RS)-(±)-2-(2-Chlorphenyl)-2-(methylamino)cyclohexan-1-on verstanden.

Besonders bevorzugt liegt (S)-Ketamin oder ein pharmazeutisch akzeptables Salz davon, insbesondere (S)-Ketamin·HCl, als einziges Stereoisomer von Ketamin vor, da die analgetische und anästhetische Potenz von (S)-Ketamin etwa dreifach höher als die der (R)-Form ist.

Der Wirkstoffgehalt in der ersten und/oder der zweiten Matrixschicht kann innerhalb relativ weiter Grenzen variieren. Als geeignet kann ein Bereich von 10 bis 60 Gew.-%, bezogen auf das Trockengewicht der jeweiligen Matrixschicht, angegeben werden. In einer Ausführungsform liegt der Anteil an Wirkstoff in der jeweiligen Matrixschicht eher im unteren Bereich, z.B. wenn der Wirkstoff einen stark unangenehmen Geschmack aufweist, der mit einer größeren Menge an geschmacksmaskierenden Mitteln kompensiert werden muss. In diesem Fall kann als geeigneter Wirkstoffanteil ein Bereich von 10 bis 40 Gew.-% angegeben werden. In einer anderen Ausführungsform liegt der Anteil an Wirkstoff in der erfindungsgemäßen Darreichungsform eher im oberen Bereich, wobei ein Gehalt von 40 bis 60 Gew.-% und insbesondere ein Gehalt von 45 bis 55 Gew.-% als besonders bevorzugt angegeben werden kann.

Besonders bevorzugt liegt (S)-Ketamin oder ein pharmazeutisch akzeptables Salz davon in einer Menge von 45 bis 55 Gew.-%, bezogen auf das Trockengewicht der ersten und/oder der zweiten Matrixschicht, in der jeweiligen Matrixschicht vor.

Der erfindungsgemäße mehrschichtige orale Dünnfilm ist ferner vorzugsweise dadurch gekennzeichnet, dass die erste und/oder die zweite Matrixschicht mindestens einen Hilfsstoff ausgewählt aus der Gruppe, umfassend Farbstoffe, Aromastoffe, Süßstoffe, Weichmacher, geschmacksmaskierende Mittel, Emulgatoren, Enhancer, pH-Regulatoren, Feuchthaltemittel, Konservierungsmittel und/oder Antioxidationsmittel, umfasst.

Jeder dieser Hilfsstoffe ist vorzugsweise jeweils in einer Menge von 0,1 bis 40 Gew.-%, vorzugsweise von 0,1 bis 30 Gew.-%, besonders bevorzugt von 0,1 bis 15 Gew.-%, ganz besonders bevorzugt von 0,1 bis 10 Gew.-% oder 0,1 bis 5 Gew.-%, bezogen auf das Gesamtgewicht der ersten und/oder der zweiten Matrixschicht, in der jeweiligen Matrixschicht enthalten.

Das mindestens eine Polyethylenglycol weist vorzugsweise ein mittleres Molekulargewicht von mindestens 8.000 bis 7.000.000 g/mol, von 8.000 g/mol bis 300.000 g/mol, vorzugsweisevon 95.000 g/mol bis 105.000 g/mol, insbesondere von etwa 100.000 g/mol, oder besonders bevorzugt von 195.000 g/mol bis 205.000 g/mol, insbesondere von etwa 200.000 g/mol, auf.

Das Molekulargewicht wird aus den nachfolgend beschriebenen Rheologiemessungen abgeleitet.

Der erfindungsgemäße mehrschichtige orale Dünnfilm ist ferner vorzugsweise dadurch gekennzeichnet, dass das mindestens eine Polyethylenglycol eine Viskosität von 30 mPa s bis 50 mPa s oder von 65 mPas bis 115 mPas, gemessen bei 25°C, aufweist.

Die angegebenen Viskositäten beziehen sich jeweils auf eine 5 Gew.-%-ige Lösung des Polyethylenglycols in Wasser und werden auf einem Brookfield Viskosimeter, Model RVF, mit Spindel No. 1 bei 50 rpm und bei einer Temperatur von 25°C gemessen.

Besonders bevorzugt werden Polyethylenglycole eingesetzt, die beispielsweise unter den Handelsnamen POLYOX WSR N-10 oder POLYOX WSR N-80 (Dow Chemical) bekannt sind.

Ferner können die Polyethylenglycole PEG 2000, PEG 4000, PEG 6000, PEG 8000, PEG 10000 oder PEG 20000 eingesetzt werden.

Der erfindungsgemäße mehrschichtige orale Dünnfilm ist ferner vorzugsweise dadurch gekennzeichnet, dass das mindestens eine Polyethylenglycol in einer Menge von 80 bis 100 Gew.-%, bezogen auf das Gesamtgewicht der mindestens einen Trennschicht, in der mindestens einen Trennschicht enthalten ist.

Der erfindungsgemäße mehrschichtige orale Dünnfilm ist ferner vorzugsweise dadurch gekennzeichnet, dass das mindestens eine Polyethylenglycol in einer Menge von 65 bis 100 Gew.-% oder 70 bis 100 Gew.-% oder 85 bis 100 Gew.-% oder 90 bis 100 Gew.-% oder 95 bis 100 Gew.-%, bezogen auf das Gesamtgewicht der mindestens einen Trennschicht, in der mindestens einen Trennschicht enthalten ist.

Der erfindungsgemäße mehrschichtige orale Dünnfilm ist ferner vorzugsweise dadurch gekennzeichnet, dass das mindestens eine Polyethylenglycol in einer Menge von 60 bis 97,5 Gew.-% oder 65 bis 97,5 Gew.-% oder 70 bis 97,5 Gew.% oder 80 bis 97, 5 Gew.-% oder 85 bis 97,5 Gew.-% oder 90 bis 97, 5 Gew.-% oder 95 bis 97,5 Gew.-%, bezogen auf das Gesamtgewicht der mindestens einen Trennschicht, in der mindestens einen Trennschicht enthalten ist.

Der erfindungsgemäße mehrschichtige orale Dünnfilm ist ferner vorzugsweise dadurch gekennzeichnet, dass das mindestens eine Polyethylenglycol in einer Menge von 60 bis 97,5 Gew.-% oder 65 bis 97,5 Gew.-% oder 70 bis 97,5 Gew.% oder 80 bis 97, 5 Gew.-% oder 85 bis 97,5 Gew.-% oder 90 bis 97, 5 Gew.-% oder 95 bis 97,5 Gew.-%, und zusätzlich 2 bis 2,5 Gew.-% mindestens eines Weichmachers, vorzugsweise Glycerol, bezogen auf das Gesamtgewicht der mindestens einen Trennschicht, in der mindestens einen Trennschicht enthalten sind.

Der erfindungsgemäße mehrschichtige orale Dünnfilm ist vorzugsweise dadurch gekennzeichnet, dass die mindestens eine Trennschicht mindestens einen Hilfsstoff ausgewählt aus der Gruppe, umfassend Farbstoffe, Aromastoffe, Süßstoffe, geschmacksmaskierende Mittel, Emulgatoren, Enhancer, pH-Regulatoren, Feuchthaltemittel, Konservierungsmittel und/oder Antioxidationsmittel, umfasst.

Jeder dieser Hilfsstoffe ist vorzugsweise jeweils in einer Menge von 0,1 bis 10 Gew.-%, vorzugsweise von 0,1 bis 5 Gew.-%, besonders bevorzugt von 0,1 bis 2,5 Gew.-%, bezogen auf das Gesamtgewicht der Trennschicht, in dieser Schicht enthalten.

Der erfindungsgemäße mehrschichtige orale Dünnfilm ist vorzugsweise dadurch gekennzeichnet, dass die mindestens eine Trennschicht mindestens einen Weichmacher, vorzugsweise Glycerol, vorzugsweise in einer Menge von 0,5 bis 5 Gew.-%, besonders bevorzugt in einer Menge von 2 bis 2,5 Gew.-%, bezogen auf das Gesamtgewicht der mindestens einen Trennschicht, enthält.

Der erfindungsgemäße mehrschichtige orale Dünnfilm ist ferner vorzugsweise dadurch gekennzeichnet, dass die erste und/oder die zweite Matrixschicht mindestens ein wasserlösliches Polymer, vorzugsweise einen Polyvinylalkohol, und Tris(hydroxymethyl)aminomethan (TRIS) enthält.

TRIS ist eine Kurzbezeichnung für Tris(hydroxymethyl)-aminomethan (THAM), auch Tromethamin, Trometamol (INN) sowie TRIS-Puffer genannt. Chemisch handelt es sich um ein primäres Amin mit drei alkoholischen Hydroxygruppen.

TRIS wird für biochemische, molekularbiologische, mikrobiologische und pharmazeutische Zwecke als Puffersubstanz verwendet. Bei einem pKs von 8,2 (bei 20 °C) besitzt TRIS eine gute Pufferkapazität zwischen pH 7,2 bis 9,0.

Eine Matrixschicht, die mindestens ein wasserlösliches Polymer, vorzugsweise einen Polyvinylalkohol, und Tris(hydroxymethyl)aminomethan (TRIS) enthält, wird im Folgenden auch als TRIS-Pufferschicht bzw. wenn Polyvinylalkohol eingesetzt wird als TRIS-PVA Schicht bezeichnet.

Insbesondere umfasst das wasserlösliche Polymer in einer solchen TRIS-Pufferschicht Polyvinylalkohol.

Eine derartige Schicht hat den Vorteil, dass sich TRIS prozentual und molar mit dem höchsten Anteil Puffer/PVA einarbeiten lässt. Bei anderen Salzen/Puffern (Phosphaten, Carbonaten, Zitronensäure etc.) kommt es hingegen zum Verklumpen bzw. Ausfallen von PVA.

Die TRIS-PVA Zusammensetzungen können gut geschäumt werden und bilden einen Film von hoher optischer Homogenität.

Polyvinylalkohole (abgekürzt mit PVA oder PVAL, gelegentlich auch mit PVOH) sind Polymere der allgemeinen Struktur 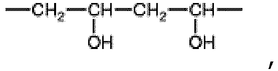 die in geringen Anteilen (etwa 2 %) auch Struktureinheiten des Typs 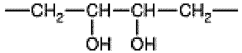 enthalten können. Sie gehören zur Gruppe der Vinylpolymere.

Handelsübliche Polyvinylalkohole, die als weiß-gelbliche Pulver oder Granulate mit Polymerisationsgraden im Bereich von etwa 500 bis 2500 (Molmassen von etwa 20000 bis 100000 g/mol) angeboten werden, haben in der Regel Hydrolysegrade von 98 bis 99 bzw. 87 bis 89 Mol-%, enthalten also noch einen Restgehalt an Acetyl-Gruppen. Charakterisiert werden die Polyvinylalkohole von Seiten der Hersteller durch Angabe des Polymerisationsgrades des Ausgangspolymeren, des Hydrolysegrades, der Verseifungszahl bzw. der Lösungsviskosität.

Gemäß der vorliegenden Erfindung sind in der TRIS-Pufferschicht Polyvinylalkohole mit einem mittleren Molekulargewicht von etwa 31.000 (PVA 4-88) bis etwa 205.000 (PVA 40-88) g/mol, besonders geeignet.

Ferner sind gemäß der vorliegenden Erfindung in der TRIS-Pufferschicht Polyvinylalkohole mit einer Viskosität von von 3,4-4,6 mPa s (PVA 4-88) bis 34-46 mPa s (PVA 40-88) in einer 40g/l wässrigen Lösung, bestimmt durch "falling ball method" (Ph.Eur. 2.2.49), besonders geeignet, oder Mischungen aus zwei oder mehr verschiedenen PVA-Typen

Der erfindungsgemäße orale Dünnfilm zeichnet sich vorzugsweise dadurch aus, dass in der TRIS-Pufferschicht Polyvinylalkohol in einer Menge von 20 bis 90 Gew.-%, vorzugsweise von 40 bis 80 Gew.-% und ganz besonders bevorzugt von 50 bis 75 Gew.-%, bezogen auf das Gesamtgewicht der TRIS-Pufferschicht, in der TRIS-Pufferschicht enthalten ist.

Der erfindungsgemäße orale Dünnfilm ist ferner vorzugsweise dadurch gekennzeichnet, dass in der TRIS-Pufferschicht Tris(hydroxymethyl)aminomethan in einer Menge von 3 bis 70 Gew.-%, vorzugsweise von 10 bis 55 Gew.-% und ganz besonders bevorzugt von 15 bis 50 Gew.-%, bezogen auf das Gesamtgewicht der TRIS-Pufferschicht, in der TRIS-Pufferschicht enthalten ist.

Vorzugsweise enthält der erfindungsgemäße mehrschichtige orale Dünnfilm, insbesondere in der TRIS-Pufferschicht keine anderen Puffersubstanzen als TRIS, insbesondere keine Phosphate, Carbonate und/oder Zitronensäure.

In der TRIS-Pufferschicht sind vorzugsweise keine anderen Polymere als PVA enthalten.

Vorzugsweise ist der erfindungsgemäße mehrschichtige orale Dünnfilm dadurch gekennzeichnet, dass die erste und/oder die zweite Matrixschicht in Form eines Hohlräume aufweisenden verfestigten Schaums vorliegt.

Durch die Hohlräume und die damit verbundene größere Oberfläche der Filme wird insbesondere der Zutritt von Wasser bzw. Speichel oder anderen Körperflüssigkeiten in das Innere der Darreichungsform erleichtert und somit die Auflösung der Darreichungsform und die Wirkstofffreisetzung beschleunigt.

Bei einem schnell resorbierenden Wirkstoff kann zudem durch die schnelle Auflösung der Matrixschicht die transmukosale Resorption verbessert werden.

Zum anderen ist die Wandstärke der genannten Hohlräume vorzugsweise gering, da diese beispielsweise verfestigte Blasen darstellen, so dass eine schnelle Auflösung oder Zerstörung dieser Hohlräume stattfindet.

Ein weiterer Vorteil dieser Ausführungsform besteht darin, dass sich trotz des vergleichsweise hohen Flächengewichts durch die Konfektionierung als Schaum eine schnellere Trocknung realisieren lässt als bei einer vergleichbaren nicht geschäumten Zusammensetzung.

Vorzugsweise ist der erfindungsgemäße mehrschichtige orale Dünnfilm dadurch gekennzeichnet, dass die Hohlräume voneinander isoliert sind, und vorzugsweise in Gestalt von Blasen vorliegen, wobei die Hohlräume mit Luft oder einem Gas, vorzugsweise mit einem inerten Gas, besonders bevorzugt mit Stickstoff, Kohlendioxid, Helium oder einem Gemisch mindestens zweier dieser Gase, gefüllt sind.

Gemäß einer anderen Ausführungsform ist vorgesehen, dass die Hohlräume miteinander in Verbindung stehen, vorzugsweise indem sie ein zusammenhängendes, die Matrix durchdringendes Kanalsystem bilden.

Vorzugsweise haben die genannten Hohlräume einen Volumenanteil von 5 bis 98 %, bevorzugt von 50 bis 80 %, bezogen auf das Gesamtvolumen der Matrixschicht. Auf diese Weise wird der vorteilhafte Effekt, die Lösung der Matrixschicht zu beschleunigen, in günstiger Weise beeinflusst.

Ferner können dem Matrix-Polymer bzw. der Polymermatrix zur Schaumbildung oder dem erhaltenen Schaum vor oder nach dem Trocknen oberflächenaktive Stoffe bzw. Tenside hinzugefügt werden, um die Stabilität des Schaums vor oder nach dem Trocknen zu verbessern.

Ein weiterer Parameter, der die Eigenschaften der erfindungsgemäßen Darreichungsform beeinflusst, ist der Durchmesser der Hohlräume oder Blasen.

Die Blasen oder Hohlräume werden vorzugsweise mit Hilfe einer Schaumaufschlagmaschine erzeugt, mit der der Durchmesser der Blasen in einem weiten Bereich, fast beliebig, eingestellt werden kann. So kann der Durchmesser der Blasen oder Hohlräume im Bereich von 0,01 bis 350 µm liegen. Besonders bevorzugt liegt der Durchmesser im Bereich von 10 und 200 µm.

Der erfindungsgemäße mehrschichtige orale Dünnfilm ist prinzipiell in der Anzahl der enthaltenen Schichten nicht beschränkt.

So sind Ausführungsformen denkbar, bei denen der mehrschichtige orale Dünnfilm zu der ersten und der zweiten Matrixschicht weitere Matrixschichten aufweist. Die obigen Definitionen gelten analog für die weiteren Matrixschichten. Diese weiteren Matrixschichten werden analog zu der ersten und zweiten Matrixschicht durch eine dazwischenliegende Trennschicht, wie oben definiert, miteinander verbunden.

In einer Ausführungsform umfasst der mehrschichtige orale Dünnfilm eine erste Matrixschicht, die Ketamin, vorzugsweise (S)-Ketamin, als pharmazeutisch aktiven Wirkstoff enthält, und eine TRIS-Pufferschicht als zweite Matrixschicht, die mit einer Trennschicht verbunden werden, wobei die erste und zweite Matrixschicht und Trennschicht wie vorstehend definiert zu verstehen sind.

In einer Ausführungsform besteht der mehrschichtige orale Dünnfilm aus einer erste Matrixschicht, die Ketamin, vorzugsweise (S)-Ketamin, als pharmazeutisch aktiven Wirkstoff enthält, und einer TRIS-Pufferschicht als zweite Matrixschicht, die mit einer Trennschicht verbunden werden, wobei die erste und zweite Matrixschicht und Trennschicht wie vorstehend definiert zu verstehen sind.

Der erfindungsgemäße orale Dünnfilm besitzt vorzugsweise eine Fläche von 0,5 cm² bis 10 cm², besonders bevorzugt von 2 cm² bis 8 cm².

Der erfindungsgemäße orale Dünnfilm ist vorzugsweise dadurch gekennzeichnet, dass das Flächengewicht des mehrschichtigen oralen Dünnfilms 10 bis 500 g/m², vorzugsweise 70 bis 400 g/m², beträgt.

Das Flächengewicht der ersten Matrixschicht, der zweiten Matrixschicht bzw. einer möglicherweise vorhandenen TRIS-Pufferschicht und jeder weiteren eventuell vorhandenen Schicht beträgt jeweils vorzugsweise mindestens 10 g/m², bevorzugter mindestens 20 g/m² oder mindestens 30 g/m² oder am meisten bevorzugt mindestens 50 g/m² oder von weniger als oder gleich 400 g/m², bevorzugter weniger als oder gleich 350 g/m² oder weniger als oder gleich 300 g/m² oder am meisten bevorzugt weniger als 250 g/m². Vorzugsweise beträgt das Flächengewicht 10 bis 400 g/m², bevorzugter 20 bis 350 g/m² oder 30 bis 300 g/m² und am meisten bevorzugt 50 bis 250 g/m².

Bevorzugt weist jede der vorhandenen Schichten jeweils eine Schichtdicke von vorzugsweise 10 µm bis 500 µm, besonders bevorzugt von 20 µm bis 300 µm, auf.

Wenn die jeweiligen Schichten in Form eines verfestigten Schaums vorliegen, so ist es bevorzugt, dass jede der als Schaum vorliegenden Schichten jeweils eine Schichtdicke von vorzugsweise 10 µm bis 3000 µm, besonders bevorzugt von 90 µm bis 2000 µm, aufweist.

Der erfindungsgemäße orale Dünnfilm kann gemäß dem Fachmann bekannten Verfahren hergestellt werden.

Der erfindungsgemäße orale Dünnfilm wird aber vorzugsweise durch ein Verfahren hergestellt, dass die folgenden Schritte umfasst:
a) Herstellen und Ausstreichen einer Lösung oder Suspension, umfassend das mindestens eine Polyethylenglycol, und anschließendes Trocknen der ausgestrichenen Lösung oder Suspension, um einen Film, umfassend das mindestens eine Polyethylenglycol zu erhalten,
b) Bereitstellen einer ersten und einer zweiten Matrixschicht, die jeweils mindestens ein Polymer umfasst,
c) Positionieren, des in Schritt a) erhaltenen Films auf der ersten Matrixschicht und Positionieren der zweiten Matrixschicht auf dem in Schritt a) erhaltenen Film, sodass der in Schritt a) erhalten Film zwischen der ersten und der zweiten Matrixschicht liegt, um einen losen Verbund zu bilden und
d) Bilden eines festen Verbundes durch Erwärmen und/oder die Applikation von Druck auf den losen Verbund aus Schritt c).

In Schritt d) wird vorzugsweise auf eine Temperatur von 30 bis 200, vorzugsweise von 50 bis 90 erwärmt.

In Schritt d) wird vorzugsweise auf einen Druck von 0,001 bar bis 20 bar, vorzugsweise von 0,01 bar bis 8 bar appliziert.

Die erste und/oder zweite Matrixschicht wird vorzugsweise durch ein Verfahren bereitgestellt, das die folgenden Schritte umfasst:
a1) Herstellen einer Lösung, Dispersion oder Schmelze, die mindestens ein Polymer umfasst,
aa1) optional Aufschäumen der Lösung, Dispersion oder Schmelze aus Schritt a1) durch Eintragen eines Gases oder Gasgemisches, durch chemische Gaserzeugung oder durch Entspannen eines gelösten Gases,
b) Ausstreichen der Lösung, Dispersion oder Schmelze aus Schritt a) bzw. der optional aufgeschäumten Lösung, Dispersion oder Schmelze aus Schritt aa1), um eine erste und/oder zweite Matrixschicht zu erhalten.

Dem Fachmann ist klar, dass der Schritt aa1) nur dann notwendig ist, wenn die erste und/oder die zweite Matrixschicht in Form eines Hohlräume aufweisenden verfestigten Schaums vorliegen soll.

Die vorliegende Erfindung betrifft ferner einen mehrschichtigen oralen Dünnfilm erhältlich nach dem vorstehend beschriebenen Verfahren.

Außerdem betrifft die vorliegende Erfindung einen mehrschichtigen oralen Dünnfilm, wie vorstehend beschrieben, oder erhältlich nach dem vorstehend beschriebenen Verfahren als Arzneimittel.

Die vorliegende Erfindung betrifft zudem einen mehrschichtigen oralen Dünnfilm, wie vorstehend beschrieben oder erhältlich nach dem vorstehend beschriebenen Verfahren, wobei Ketamin, vorzugsweise S-Ketamin, bzw. ein pharmazeutisch akzeptables Salz davon, als pharmazeutisch aktiver Wirkstoff in der ersten und/oder der zweiten Matrixschicht eingesetzt wird, zur Verwendung in der Behandlung von Schmerzen und/oder Depressionen, insbesondere zur Reduzierung des Suizidrisikos, und/oder zur Verwendung als Allgemeinanästhetikum, vorzugsweise zur Einleitung und Durchführung einer Vollnarkose oder als Ergänzung bei Regionalanästhesien und/oder als Analgetikum.

Die vorstehend für den erfindungsgemäßen mehrschichtigen oralen Dünnfilm aufgeführten bevorzugten Ausführungsformen gelten auch für das erfindungsgemäße Verfahren, den durch dieses Verfahren erhaltenen mehrschichtigen oralen Dünnfilm und dessen Verwendung als Arzneimittel.

Die Erfindung wird nachfolgend anhand von nicht beschränkenden Beispielen näher erläutert.

### Beispiele:

### Beispiel 1:

Herstellung einer ersten Matrixschicht mit PolyOx-Deckschicht:

Beispielformulierung aus einer PolyOx-Schicht (nicht geschäumt) und einer S-Ketamin haltigen ersten Matrixschicht (in Form eines Schaums) durch Mehrfachbeschichtung:

**Tabelle 1:**

| **Material** | **1 [Gew.-%]** |
|---|---|
| (S)-Ketamin HCl | 50.00 % |
| PVA 4-88 | 41.70 % |
| PVA 40-88 | -- |
| Saccharin Na | 1.00 % |
| Sucralose | 2.00 % |
| Glycerol | 2.30 % |
| Geschmacksstoff 1 | 1.00 % |
| Geschmacksstoff 2 | 2.00 % |
| Polyox WSR N10 | 97.50 % |
| Glycerol | 2.3 % |
| FD&C Red 40 (Farbstoff) | 0.20 % |
| **Lösungsmittel** | Aqua purificata |
| **Flächengewicht (trocken)** | 1. Matrixschicht: |
| | 118.7 g/cm² |
| | Trennschicht: 100 |
| | g/cm² |
| **Beschichtungsgewicht (incl. res. Water)** | 1. Matrixschicht: |
| | 121.9 g/cm² |
| | Trennschicht |
| | 101.5 g/cm² |

### Herstellung:

Die Formulierung 1 ist eine Zweischichtformulierung, wobei eine erste Polyox-Schicht eine wirkstofffreie Trennschicht bildet, die zuerst hergestellt wird. Diese Schicht wird anschließend mit einer wirkstoffhaltigen Matrixschicht überzogen, wobei 50 % (S)-Ketamin·HCl in einer Polyvinylalkohol (PVA) 4-88 Schaummatrix enthalten sind. Das Trockenflächengewicht für die Polyox-Trennschicht ist mit 100,0 g/m² definiert, was zu einem Beschichtungsgewicht von 101,5 g/m² inkl. 1,5 % Restwasser führt. Das Trockenflächengewicht für die wirkstoffhaltige Matrixschicht ist definiert als 118,7 g/m², was zu einem Beschichtungsgewicht von 123,6 g/m² inkl. 4 % Restwasser (Erwartungswert) führt.

### Beispiel 2:

Beispielformulierung aus einer PolyOx-Trennschicht (nicht geschäumt) und einer S-Ketamin haltigen ersten Matrixschicht (geschäumt) und einer TRIS-Pufferschicht als zweite Matrixschicht, verbunden durch Laminieren:

**Tabelle 2:**

| **S-Ketamin haltige 1. Matrixschicht** | **2 [Gew.-%]** |
|---|---|
| (S)-Ketamin HCl | 50,00 % |
| PVA 4-88 | 41,70 % |
| Saccharin Na | 1,00 % |
| Sucralose | 2,00 % |
| Glycerol | 2,30 % |
| Geschmacksstoff 1 | 1,00 % |
| Geschmacksstoff 2 | 2,00 % |
| **Lösungsmittel** | Aqua purificata |
| Flächengewicht (trocken) | 117,3 g/m" |
| | |

| **PolyOx-Trennschicht** | **3 [Gew.-%]** |
|---|---|
| Polyox WSR N10 | 97,50 % |
| Glycerol | 2,3 % |
| FD&C Red 40 (Farbstoff) | 0,20 % |
| **Lösungsmittel** | Aqua purificata |
| Flächengewicht (trocken) | 98,2 g/m" |
| | |

| **2. Matrixschicht (TRIS-Pufferschicht)** | **4 [Gew.-%]** |
|---|---|
| PVA 4-88 | 36,15 |
| PVA 40-88 | 36,15 |
| TRIS | 20,00 |
| Saccharin Na | 1,00 |
| Sucralose | 2,00 |
| Glycerol | 4,50 |
| FD&C Red No. 40 (Farbstoff) | 0,20 |
| Lösungsmittel | Aqua purificata |
| Area weight (trocken) | 192,8 g/m² |
| BEschichtungsgewicht (incl. res. Water) | 200,6 g/m² |

Die drei Schichten wurden separat hergestellt und die erhaltenen Laminate auf 70 °C erhitzt und durch laminieren verbunden. Nach dem Abkühlen waren die Laminate fest verbunden.

### pH-Messung

Es wurde der pH-Wert der einzelnen Schichten aus Tabelle 2, sowie der Verbünde aus diesen Schichten gemessen.

**Tabelle 3:**

| **Probe** | **Temperatur Medium** | **pH** |
|---|---|---|
| TRIS-Schicht 4 | 33,3°C 2 mL Wasser | 8,54 |
| Kea-Schicht 2 | 33,4°C 2 mL Wasser | 5,71 |
| PolyOx-Schicht 3 | 33,3 °C 2 mL Wasser | 7,58 |
| TRIS/Polyox/Kea (3-Schicht OTF) | 33,6°C 2 mL Wasser | 7,24 |
| TRIS-Schicht 4 | 30,3°C 2 mL Glandosane¹ | 8,48 |
| Kea-Schicht 2 | 33,7°C 2 mL Glandosane¹ | 5,46 |
| PolyOx-Schicht 3 | 32,6 2 mL Glandosane¹ | 6,02 |
| TRIS/Polyox/Kea (3-Schicht OTF) | 31,8°C 2 mL Glandosane¹ | 7,06 |

| | | |
|---|---|---|
| ¹:Glandosane = künstlicher Speichel | | |

### Beispiel 3:

Es wurde die Migration von Stoffen zwischen einzelnen Schichten eines mehrschichtigen oralen Dünnfilms untersucht, wobei die einzelnen Schichten über verschiedene Methoden miteinander verbunden wurden.
1. Zweischichtiger oraler Dünnfilm (OTF1) - Verbindung der Schichten durch eine Naht.

Die beiden Schichten des OTF1 weisen eine Zusammensetzung gemäß den folgenden Tabellen 4 bzw. 5 auf.

**Tabelle 4:**

| **Material** | **[Gew.-%]** |
|---|---|
| PVA 4-88 | 39,50 |
| Saccharin Na | 1,0 |
| Sucralose | 2,0 |
| Raspberry flavor | 2,0 |
| Masking flavor | 1,0 |
| Glycerol | 4,5 |
| S-Ketamin HCl | 50,0 |
| Flächengewicht | 121,9 g/m² |

**Tabelle 5:**

| **Material** | **[Gew.-%]** |
|---|---|
| PVA 4-88 | 36,15 |
| PVA 40-88 | 36,15 |
| TRIS | 20,00 |
| Saccharin Na | 1,00 |
| Sucralose | 2,00 |
| Glycerol | 4,50 |
| FD&C Red No. 40 | 0,20 |
| Flächengewicht | 200,6 g/m² |

Es wurden zwei einzelne Schichten mit einer Zusammensetzung gemäß den Tabellen 4 bzw. 5 und einer Fläche von 2,72 cm² hergestellt und mit einem PVA-Faden von 0,2 mm Stärke (Vis Extrusion GmbH, Hohberg, Deutschland) von Hand vernäht.

2. Zweischichtiger oraler Dünnfilm (OTF2) - Verbindung der Schichten durch eine Siegelung.

Die beiden Schichten des OTF2 weisen eine Zusammensetzung gemäß den obigen Tabellen 4 bzw. 5 auf.

Es wurden zwei einzelne Schichten mit einer Zusammensetzung gemäß den Tabellen 4 bzw. 5 und einer Fläche von 2,72 cm² hergestellt und durch eine Siegelung verbunden. Dazu wurde ein Handsiegelgerät (Polystar 100 GEW bei Level 9 und mit 850 µm Fühlerlehrenabstand) eingesetzt.

3. Dreischichtiger oraler Dünnfilm (OTF3) - Verbindung der Schichten durch eine PolyOx-Zwischenschicht.

Die drei Schichten des OTF3 weisen eine Zusammensetzung gemäß der obigen Tabelle 5 und den folgenden Tabellen 6 und 7 auf.

**Tabelle 6:**

| **Material** | **Schaumformulierung [Gew.-%]** |
|---|---|
| PVA 4-88 | 41,70 |
| Saccharin Na | 1,00 |
| Sucralose | 2,00 |
| Glycerol | 2,30 |
| Masking Flavor PHL-131985 | 1,00 |
| Raspberry Flavor PHL-097336 | 2,00 |
| S-Ketamin HCl | 50,00 |
| Flächengewicht | 121,9 g/m² |

**Tabelle 7:**

| **Material** | **[Gew.-%]** |
|---|---|
| Polyethylenoxid MW 100,000 | 97,50 |
| Glycerol | 2,30 |
| FD&C Red No. 40 | 0,20 |
| Flächengewicht | 101,5 g/m² |

Es wurde ein dreischichtiger oraler Dünnfilm hergestellt, wobei die drei Schichten eine Zusammensetzung gemäß der obigen Tabelle 5 und den Tabellen 6 und 7 aufweisen.

Dazu wurden die einzelnen Schichten so zu einem losen Verbund angeordnet, dass die PolyOx-Schicht (Tabelle 7) in der Mitte liegt. Durch Erwärmen des losen Verbundes auf etwa 70°C wird das PolyOx angeschmolzen und die Schichten des losen Verbundes verbinden sich.

Die OTFs aller drei Formulierungen wurden in COC-Beuteln von 60x60 mm versiegelt und in einem 40°C-Laborschrank gelagert. Die Migrationstests wurden nach 3 und 6 Wochen Lagerung mit n=3 geplant. Aufgrund eines Missverständnisses wurden alle 6 OTFs der drei Formulierungen nach 3 Wochen aus der Lagerung entfernt, aber nur 3 OTFs wurden analysiert. Somit war es möglich, die verbliebenen 3 OTFs nach 3 Wochen Lagerung bei Raumtemperatur in den 40°C-Schrank wiederherzustellen. Nach weiteren 3 Wochen bei 40°C wurde der 6-wöchige Testpunkt (3 Wochen bei 40°C + 3 Wochen RT + 3 Wochen 40°C) analysiert.

Es wurde die Migration von Stoffen zwischen den einzelnen Schichten untersucht.

**Tabelle 8: pH-Werte (n=3) in den ungelagerten wirkstoffhaltigen Schichten**

| | | Nicht gelagert | | | |
|---|---|---|---|---|---|
| | | Probe 1 | Probe 2 | Probe 3 | Ø |
| OTF1 | genäht | 5,75 | 5,75 | 5,75 | 5,75 |
| OTF2 | gesiegelt | 5,75 | 5,75 | 5,75 | 5,75 |
| OTF3 | PolyOx | 6,12 | 6,13 | 6,14 | 6,13 |

**Tabelle 9: pH-Werte (n=3) in den wirkstoffhaltigen Schichten**

| | | 3 Wochen | | | | 6 Wochen | | | |
|---|---|---|---|---|---|---|---|---|---|
| | | Probe 1 | Probe 2 | Probe 3 | Ø | Probe 1 | Probe 2 | Probe 3 | Ø |
| OTF1 | genäht | 6,47 | 6,43 | 6,48 | 6,46 | 6,53 | 6,46 | 6,45 | 6,48 |
| OTF2 | gesiegelt | 6,44 | 6,54* | 6,54* | 6,51 | 6,44* | 6,42* | 6,45* | 6,44 |
| OTF3 | PolyOx | 6,23 | 6,19 | 6,18 | 6,20 | 6,32 | 6,29 | 6,28 | 6,30 |

| | | | | | | | | | |
|---|---|---|---|---|---|---|---|---|---|
| * Siegelnaht verworfen | | | | | | | | | |

**Tabelle 10: pH-Wert Unterschiede in den wirkstoffhaltigen Schichten**

| | | Lagerung | | |
|---|---|---|---|---|
| | | Nicht gelagert | 3 Wochen | 6 Wochen |
| OTF1 | genäht | --- | +0,71 | +0,73 |
| OTF2 | gesiegelt | --- | +0,76* | +0,69* |
| OTF3 | PolyOx | --- | +0,07 | +0,17 |

| | | | | |
|---|---|---|---|---|
| * Siegelnaht verworfen | | | | |

**Tabelle 11: Wirkstoffgehalt in den pufferhaltigen Schichten**

| | | 3 Wochen | | | 6 Wochen | | |
|---|---|---|---|---|---|---|---|
| | | Gehalt mg | % LC | Gew.-% | Gehalt mg | % LC | Gew.% |
| OTF1 | genäht | 1.0 | 6.2 | 1.9 | 1.0 | 6.1 | 1.8 |
| OTF2 | gesiegel t | 0.9 | 5.3 | 2.0 | 0.4 | 2.7 | 1.1 |
| OTF3 | PolyOx | 0.6 | 3.8 | 1.0 | 0.8 | 4.8 | 1.4 |

| | | | | | | | |
|---|---|---|---|---|---|---|---|
| LC = lable claim 16.1mg/OTF n=3 | | | | | | | |

Die niedrigsten pH-Werte und Wirkstoffgehalte wurden in der PolyOx-Formulierung gefunden. Daher scheint die Formulierung OTF3 die beste Möglichkeit zu sein, die zwei Schichten mit einer geringeren Migration von Wirkstoff zu kombinieren.

## Patentansprüche

1. Mehrschichtiger oraler Dünnfilm, umfassend eine erste und eine zweite Matrixschicht, die jeweils mindestens ein Polymer enthalten, und eine zwischen der ersten und der zweiten Matrixschicht befindliche Trennschicht, wobei die Trennschicht mindestens ein Polyethylenglycol mit einem Molekulargewicht von 8000 g/mol bis 7000000 g/mol in einer Menge von 60 bis 100 Gew.-% umfasst.

2. Mehrschichtiger oraler Dünnfilm gemäß Anspruch 1, wobei die erste und/oder die zweite Matrixschicht mindestens ein wasserlösliches Polymer umfasst.

3. Mehrschichtiger oraler Dünnfilm gemäß Anspruch 2, wobei das mindestens eine wasserlösliche Polymer ausgewählt ist aus der Gruppe, umfassend Stärke und Stärkederivate, Dextrane, Cellulosederivate, wie Carboxymethylcellulose, Hydroxypropylcellulose, Hydroxyethylcellulose, Hydroxypropylmethylcellulose, Hydroxypropylethylcellulose, Natriumcarboxymethylcellulose, Ethyl- oder Propylcellulose, Polyacrylsäure, Polyacrylate, Polyvinylpyrrolidone, Vinvlpyrrolidon/Vinylacetat-Copolymere, Polyvinylalkohole, Polyethylenoxidpolymere, Polyacrylamide, Polyethylenglycole, Gelatine, Collagen, Alginaten, Pektin, Pullulan, Traganth, Chitosan, Alginsäure, Arabinogalaktan, Galaktomannan, Agar, Agarose, Carrageen und natürlichen Gummen.

4. Mehrschichtiger oraler Dünnfilm gemäß irgendeinem der vorhergehenden Ansprüche, wobei die erste und/oder die zweite Matrixschicht mindestens einen pharmazeutisch aktiven Wirkstoff umfassen, wobei der mindestens eine pharmazeutisch aktive Wirkstoff vorzugsweise ausgewählt ist aus der Gruppe, umfassend die Wirkstoffklassen der Analgetika, Hormone, Hypnotika, Sedativa, Antiepileptika, Weckamine, Psychoneurotropika, Neuro-Muskelblocker, Antispasmodika, Antihistaminika, Antiallergika, Cardiotonika, Antiarrhythmika, Diuretika, Hypotensiva, Vasopressoren, Antidepressiva, Antitussiva, Expectorantia, Thyroidhormone, Sexualhormone, Antidiabetika, Antitumor-Wirkstoffe, Antibiotika, Chemotherapeutika und Narcotika, wobei der mindestens eine pharmazeutisch aktive Wirkstoff vorzugsweise Ketamin, besonders bevorzugt (S)-Ketamin, umfasst.

5. Mehrschichtiger oraler Dünnfilm gemäß irgendeinem der vorhergehenden Ansprüche, wobei die erste und/oder die zweite Matrixschicht ferner mindestens einen Hilfsstoff ausgewählt aus der Gruppe, umfassend Farbstoffe, Aromastoffe, Süßstoffe, Weichmacher, geschmacksmaskierende Mittel, Emulgatoren, Enhancer, pH-Regulatoren, Feuchthaltemittel, Konservierungsmittel und/oder Antioxidationsmittel, umfasst.

6. Mehrschichtiger oraler Dünnfilm gemäß irgendeinem der vorhergehenden Ansprüche, wobei das mindestens eine Polyethylenglycol ein mittleres Molekulargewicht von 8000 g/mol bis 300000 g/mol, vorzugsweise von 95000 g/mol bis 105000 g/mol, insbesondere von etwa 100000 g/mol, oder besonders bevorzugt von 195.000 g/mol bis 205.000 g/mol, insbesondere von 200.000 g/mol, aufweist.

7. Mehrschichtiger oraler Dünnfilm gemäß irgendeinem der vorhergehenden Ansprüche, wobei das mindestens eine Polyethylenglycol eine Viskosität von 30 mPa s bis 50 mPa s, gemessen in 5 Gew.-%-iger wässriger Lösung bei 25°C, aufweist.

8. Mehrschichtiger oraler Dünnfilm gemäß irgendeinem der vorhergehenden Ansprüche, wobei das mindestens eine Polyethylenglycol in einer Menge von 80 bis 100 Gew.-%, bezogen auf das Gesamtgewicht der Trennschicht, in der mindestens einen Trennschicht enthalten ist.

9. Mehrschichtiger oraler Dünnfilm gemäß irgendeinem der vorhergehenden Ansprüche, wobei die erste und/oder die zweite Matrixschicht mindestens ein wasserlösliches Polymer, vorzugsweise einen Polyvinylalkohol, und Tris(hydroxymethyl)aminomethan enthält.

10. Mehrschichtiger oraler Dünnfilm gemäß Anspruch 9, wobei das wasserlösliche Polymer, vorzugsweise der Polyvinylalkohol, in einer Menge von 20 bis 90 Gew.-%, bezogen auf das Gesamtgewicht der ersten und/oder zweiten Matrixschicht, in dieser Schicht enthalten ist.

11. Mehrschichtiger oraler Dünnfilm gemäß Anspruch 9 oder 10, wobei Tris(hydroxymethyl)aminomethan in einer Menge von 3 bis 70 Gew.-%, bezogen auf das Gesamtgewicht der ersten und/oder zweiten Matrixschicht, in dieser Schicht enthalten ist.

12. Mehrschichtiger oraler Dünnfilm gemäß irgendeinem der vorhergehenden Ansprüche, wobei die erste und/oder die zweite Matrixschicht in Form eines Hohlräume aufweisenden verfestigten Schaums vorliegt.

13. Mehrschichtiger oraler Dünnfilm gemäß Anspruch 12, wobei die Hohlräume voneinander isoliert sind und vorzugsweise in Gestalt von Blasen vorliegen, wobei die Hohlräume mit Luft oder einem Gas, vorzugsweise mit einem inerten Gas, besonders bevorzugt mit Stickstoff, Kohlendioxid, Helium oder einem Gemisch mindestens zweier dieser Gase, gefüllt sind.

14. Mehrschichtiger oraler Dünnfilm gemäß 12 oder 13, wobei die Hohlräume miteinander in Verbindung stehen und vorzugsweise ein die jeweilige Matrixschicht durchdringendes Kanalsystem bilden.

15. Mehrschichtiger oraler Dünnfilm gemäß irgendeinem der Ansprüche 12 bis 14, wobei die Hohlräume einen Volumenanteil von 5 bis 98 %, bevorzugt von 50 bis 80 %, bezogen auf das Gesamtvolumen der jeweiligen Matrixschicht, in der jeweiligen Matrixschicht aufweisen.

16. Verfahren zur Herstellung eines mehrschichtigen oralen Dünnfilm gemäß irgendeinem der Ansprüche 1 bis 15, umfassend die Schritte
a) Herstellen und Ausstreichen einer Lösung oder Suspension, umfassend das mindestens eine Polyethylenglycol, und anschließendes Trocknen der ausgestrichenen Lösung oder Suspension, um einen Film, umfassend das mindestens eine Polyethylenglycol zu erhalten,
b) Bereitstellen einer ersten und einer zweiten Matrixschicht, die jeweils mindestens ein Polymer umfasst;
c) Positionieren, des in Schritt a) erhaltenen Films auf der ersten Matrixschicht und Positionieren der zweiten Matrixschicht auf dem in Schritt a) erhaltenen Film, sodass der in Schritt a) erhalten Film zwischen der ersten und der zweiten Matrixschicht liegt, um einen losen Verbund zu bilden und
d) Bilden eines festen Verbundes durch Erwärmen und/oder die Applikation von Druck auf den losen Verbund aus Schritt c).

17. Verfahren gemäß Anspruch 16, wobei die erste und/oder zweite Matrixschicht durch ein Verfahren bereitgestellt wird, das die folgenden Schritte umfasst:
a1) Herstellen einer Lösung, Dispersion oder Schmelze, die mindestens ein Polymer umfasst,
aa1) optional Aufschäumen der Lösung, Dispersion oder Schmelze aus Schritt a1) durch Eintragen eines Gases oder Gasgemisches, durch chemische Gaserzeugung oder durch Entspannen eines gelösten Gases,
b) Ausstreichen der Lösung, Dispersion oder Schmelze aus Schritt a) bzw. der optional aufgeschäumten Lösung, Dispersion oder Schmelze aus Schritt aa1), um eine erste und/oder zweite Matrixschicht zu erhalten.

18. Mehrschichtiger oraler Dünnfilm gemäß irgendeinem der Ansprüche 1 bis 15 oder eines mehrschichtigen oralen Dünnfilms erhältlich gemäß dem Verfahren nach Anspruch 16 oder 17 zur Verwendung als Arzneimittel.

## Claims

1. A multi-layer oral thin film comprising a first and a second matrix layer, which each contain at least one polymer, and a separation layer located between the first and the second matrix layer, wherein the separation layer comprises at least one polyethylene glycol with a molecular weight of from 8,000 g/mol to 7,000,000 g/mol in an amount of from 60 to 100 wt.%.

2. The multi-layer oral thin film according to claim 1, wherein the first and/or the second matrix layer comprises at least one water-soluble polymer.

3. The multi-layer oral thin film according to claim 2, wherein the at least one water-soluble polymer is selected from the group comprising starch and starch derivatives, dextrans, cellulose derivatives, such as carboxymethyl cellulose, hydroxypropyl cellulose, hydroxyethyl cellulose, hydroxypropyl methylcellulose, hydroxypropyl ethyl cellulose, sodium carboxymethyl cellulose, ethyl or propyl cellulose, polyacrylic acids, polyacrylates, polyvinylpyrrolidones, vinyl pyrrolidone/vinyl acetate copolymers, polyvinyl alcohols, polyethylene oxide polymers, polyacrylamides, polyethylene glycols, gelatines, collagen, alginates, pectin, pullulan, tragacanth, chitosan, alginic acid, arabinogalactan, galactomannan, agar, agarose, carrageenan, and natural gums.

4. The multi-layer oral thin film according to any one of the preceding claims, wherein the first and/or the second matrix layer comprise at least one pharmaceutically active agent, wherein the at least one pharmaceutically active agent is preferably selected from the group comprising the active agent classes of analgesics, hormones, hypnotics, sedatives, antiepiletics, analeptics, psychoneurotropic drugs, neuro-muscle blockers, antspasmodics, antihistamines, antiallergics, cardiotonics, antiarrhythmics, diuretics, hypotensives, vasopressors, antidepressants, antitussives, expectorants, thyroid hormones, sexual hormones, antidiabetics, antitumour active agents, antibiotics, chemotherapeutics and narcotics, wherein the at least one pharmaceutically active agent preferably comprises ketamine, especially preferably (S)-ketamine.

5. The multi-layer oral thin film according to any one of the preceding claims, wherein the first and/or the second matrix layer further comprises at least one auxiliary substance selected from the group comprising colouring agents, flavourings, sweeteners, plasticisers, taste-masking agents, emulsifiers, enhancers, pH regulators, humectants, preservatives and/or antioxidants.

6. The multi-layer oral thin film according to any one of the preceding claims, wherein the at least one polyethylene glycol has a mean molecular weight of from 8,000 g/mol to 300,000 g/mol, preferably from 95,000 g/mol to 105,000 g/mol, especially of about 100,000 g/mol, or especially preferably from 195,000 g/mol to 205,000 g/mol, especially of 200,000 g/mol.

7. The multi-layer oral thin film according to any one of the preceding claims, wherein the at least one polyethylene glycol has a viscosity of from 30 mPa s to 50 mPa s, measured in 5 wt.% aqueous solution at 25°C.

8. The multi-layer oral thin film according to any one of the preceding claims, wherein the at least one polyethylene glycol is contained in the at least one separation layer in an amount of from 80 to 100 wt.%, in relation to the total weight of the separation layer.

9. The multi-layer oral thin film according to any one of the preceding claims, wherein the first and/or the second matrix layer contains at least one water-soluble polymer, preferably a polyvinyl alcohol and tris(hydroxymethyl)aminomethane.

10. The multi-layer oral thin film according to claim 9, wherein the water-soluble polymer, preferably the polyvinyl alcohol, is contained in the first and/or second matrix layer in an amount of from 20 to 90 wt.%, in relation to the total weight of this layer.

11. The multi-layer oral thin film according to claim 9 or claim 10, wherein tris(hydroxymethyl)aminomethane is contained in the first and/or second matrix layer in an amount of from 3 to 70 wt.%, in relation to the total weight of this layer.

12. The multi-layer oral thin film according to any one of the preceding claims, wherein the first and/or the second matrix layer is present in the form of a solidified foam that has voids.

13. The multi-layer oral thin film according to claim 12, wherein the voids are isolated from one another and are preferably present in the form of bubbles, wherein the voids are filled with air or a gas, preferably with an inert gas, especially preferably with nitrogen, carbon dioxide, helium or a mixture of at least two of these gases.

14. The multi-layer oral thin film according to claim 12 or claim 13, wherein the voids are connected to one another and preferably form a channel system penetrating the particular matrix layer.

15. The multi-layer oral thin film according to any one of claims 12 to 14, wherein the voids in the particular matrix layer have for a volume fraction of from 5 to 98%, preferably from 50 to 80%, in relation to the total volume of the particular matrix layer.

16. A method for producing a multi-layer oral thin film according to any one of claims 1 to 15, comprising the steps of:
a) producing and spreading a solution or suspension comprising the at least one polyethylene glycol, and then drying the spread solution or suspension in order to obtain a film comprising the at least one polyethylene glycol,
b) providing a first and a second matrix layer, which each comprise at least one polymer;
c) positioning the film obtained in step a) on the first matrix layer and positioning the second matrix layer on the film obtained in step a) so that the film obtained in step a) lies between the first and the second matrix layer in order to form a loose grouping, and
d) forming a firm bond by heating and/or by applying pressure to the loose grouping from step c).

17. The method according to claim 16, wherein the first and/or second matrix layer is provided by a method comprising the following steps:
a1) producing a solution, dispersion or melt comprising at least one polymer,
aa1) optionally foaming the solution, dispersion or melt from step a1) by introducing a gas or gas mixture by chemical gas generation or by expansion of a dissolved gas,
b) spreading the solution, dispersion or melt from step a) or the optionally foamed solution, dispersion or melt from step aa1) in order to obtain a first and/or second matrix layer.

18. Multi-layer oral thin film according to any one of claims 1 to 15 or of a multi-layer oral thin film obtained by the method according to claim 16 or 17 for the use as a medicament.

## Revendications

1. Film mince oral multicouche, comprenant une première et une deuxième couche de matrice, qui contiennent chacune au moins un polymère, et une couche de séparation se trouvant entre la première et la deuxième couche de matrice, dans lequel la couche de séparation comprend au moins un polyéthylène glycol avec un poids moléculaire de 8000 g/mol à 7 000 000 g/mol en une quantité de 60 à 100 % en poids.

2. Film mince oral multicouche selon la revendication 1, dans lequel la première et/ou la deuxième couche de matrice comprend au moins un polymère soluble dans l'eau.

3. Film mince oral multicouche selon la revendication 2, dans lequel l'au moins un polymère soluble dans l'eau est choisi dans le groupe comprenant l'amidon et les dérivés d'amidon, les dextranes, les dérivés de cellulose, tels que la carboxyméthylcellulose, l'hydroxypropylcellulose, l'hydroxyéthylcellulose, l'hydroxypropylméthylcellulose, l'hydroxypropyléthylcellulose, la carboxyméthylcellulose de sodium, l'éthylcellulose ou la propylcellulose, l'acide polyacrylique, les polyacrylates, la polyvinylpyrrolidone, les copolymères de vinylpyrrolidone/acétate de vinyle, les alcools polyvinyliques, les polymères d'oxyde de polyéthylène, les polyacrylamides, les polyéthylènes glycol, la gélatine, le collagène, les alginates, la pectine, le pullulane, la gomme adragante, le chitosan, l'acide alginique, l'arabinogalactane, le galactomannane, la gélose, l'agarose, le carraghénane et les gommes naturelles.

4. Film mince oral multicouche selon l'une quelconque des revendications précédentes, dans lequel la première et/ou la deuxième couche de matrice comprennent au moins un principe actif pharmaceutique, dans lequel l'au moins un principe actif pharmaceutique est de préférence choisi dans le groupe comprenant les classes de principes actifs des analgésiques, des hormones, des hypnotiques, des sédatifs, des antiépileptiques, des weckamines, des psychoneurotropes, des bloqueurs neuromusculaires, des antispasmodiques, des antihistaminiques, des antiallergiques, des cardiotoniques, des antiarythmiques, des diurétiques, des hypotenseurs, des vasopresseurs, des antidépresseurs, des antitussifs, des expectorants, des hormones thyroïdiennes, des hormones sexuelles, des antidiabétiques, des principes actifs antitumoraux, des antibiotiques, des chimiothérapeutiques et des narcotiques, dans lequel l'au moins un principe actif pharmaceutique comprend de préférence la kétamine, de manière particulièrement préférée la (S)-kétamine.

5. Film mince oral multicouche selon l'une quelconque des revendications précédentes, dans lequel la première et/ou la deuxième couche de matrice comprend en outre au moins un excipient choisi dans le groupe comprenant les colorants, les arômes, les édulcorants, les plastifiants, les agents de masquage de goût, les émulsifiants, les exhausteurs, les régulateurs de pH, les humectants, les conservateurs et/ou les antioxydants.

6. Film mince oral multicouche selon l'une quelconque des revendications précédentes, dans lequel l'au moins un polyéthylène glycol présente un poids moléculaire moyen de 8000 g/mol à 300 000 g/mol, de préférence de 95 000 g/mol à 105 000 g/mol, en particulier d'environ 100 000 g/mol, ou de manière particulièrement préférée de 195 000 g/mol à 205 000 g/mol, en particulier de 200 000 g/mol.

7. Film mince oral multicouche selon l'une quelconque des revendications précédentes, dans lequel l'au moins un polyéthylène glycol présente une viscosité de 30 mPa s à 50 mPa s, mesurée dans une solution aqueuse à 5 % en poids à 25 °C.

8. Film mince oral multicouche selon l'une quelconque des revendications précédentes, dans lequel l'au moins un polyéthylène glycol est contenu en une quantité de 80 à 100 % en poids, par rapport au poids total de la couche de séparation, dans l'au moins une couche de séparation.

9. Film mince oral multicouche selon l'une quelconque des revendications précédentes, dans lequel la première et/ou la deuxième couche de matrice contient au moins un polymère soluble dans l'eau, de préférence un alcool polyvinylique, et du tris(hydroxyméthyl)aminométhane.

10. Film mince oral multicouche selon la revendication 9, dans lequel le polymère soluble dans l'eau, de préférence l'alcool polyvinylique, est contenu en une quantité de 20 à 90 % en poids, par rapport au poids total de la première et/ou deuxième couche de matrice, dans cette couche.

11. Film mince oral multicouche selon la revendication 9 ou 10, dans lequel du tris(hydroxyméthyl)aminométhane est contenu en une quantité de 3 à 70 % en poids, par rapport au poids total de la première et/ou deuxième couche de matrice, dans cette couche.

12. Film mince oral multicouche selon l'une quelconque des revendications précédentes, dans lequel la première et/ou la deuxième couche de matrice est présente sous forme d'une mousse solidifiée présentant des cavités.

13. Film mince oral multicouche selon la revendication 12, dans lequel les cavités sont isolées les unes des autres et se présentent de préférence sous la forme de bulles, dans lequel les cavités sont remplies d'air ou d'un gaz, de préférence d'un gaz inerte, de manière particulièrement préférée d'azote, de dioxyde de carbone, d'hélium ou d'un mélange d'au moins deux de ces gaz.

14. Film mince oral multicouche selon 12 ou 13, dans lequel les cavités sont reliées entre elles et forment de préférence un système de canaux pénétrant la couche de matrice respective.

15. Film mince oral multicouche selon l'une quelconque des revendications 12 à 14, dans lequel les cavités présentent un pourcentage volumique de 5 à 98 %, de préférence de 50 à 80 %, par rapport au volume total de la couche de matrice respective, dans la couche de matrice respective.

16. Procédé pour la fabrication d'un film mince oral multicouche selon l'une quelconque des revendications 1 à 15, comprenant les étapes
a) de préparation et d'étalement d'une solution ou suspension, comprenant l'au moins un polyéthylène glycol, puis de séchage de la solution ou suspension étalée pour obtenir un film comprenant l'au moins un polyéthylène glycol,
b) de fourniture d'une première et d'une deuxième couche de matrice, qui comprend chacune au moins un polymère ;
c) de positionnement du film obtenu à l'étape a) sur la première couche de matrice et de positionnement de la deuxième couche de matrice sur le film obtenu à l'étape a), de sorte que le film obtenu à l'étape a) se trouve entre la première et la deuxième couche de matrice, pour former un ensemble lâche et
d) de formation d'un ensemble solide par chauffage et/ou l'application de pression sur l'ensemble lâche de l'étape c) .

17. Procédé selon la revendication 16, dans lequel la première et/ou deuxième couche de matrice est fournie par un procédé qui comprend les étapes suivantes :
a1) la préparation d'une solution, dispersion ou masse fondue, qui comprend au moins un polymère,
aa1) éventuellement le moussage de la solution, dispersion ou masse fondue de l'étape a1) par introduction d'un gaz ou mélange de gaz, par production chimique de gaz ou par détente d'un gaz dissous,
b) l'étalement de la solution, dispersion ou masse fondue de l'étape a) ou de la solution, dispersion ou masse fondue éventuellement moussée de l'étape aa1), pour obtenir une première et/ou deuxième couche de matrice.

18. Film mince oral multicouche selon l'une quelconque des revendications 1 à 15 ou d'un film mince oral multicouche pouvant être obtenu selon le procédé selon la revendication 16 ou 17 destiné à être utilisé comme médicament.
